# EUROPEAN PATENT APPLICATION

(11) **EP 2 239 957 A1**
(43) Date of publication of application: **13.10.2010**
(21) Application number: 10001787.0
(22) Date of filing: 22.02.2010
(51) Int. Cl.: H04R 1/10, A61F 11/08, H04R 25/00

(54) **Device and method to fully or partially occlude the external ear**

(30) Priority: 06.04.2009 GB 0905900
(71) Applicant: Bateson, Tobias Murray, Truro Cornwall TR1 1HW (GB)
(72) Inventor: Bateson, Tobias Murray, Truro Cornwall TR1 1HW (GB)

(57) **Abstract**

A device which totally or partially occludes the external ear which can be made in a one-stage process from a plastic with a low melting point, using the ear as a mould. The device may be used as earpieces for stethoscopes and electronic audio equipment or as earplugs.

## Description

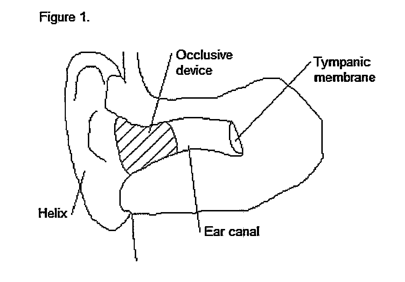

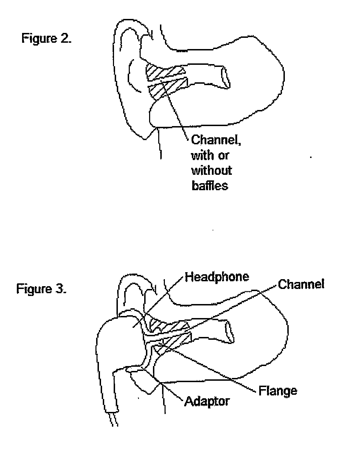

The proposed device consists of a plug of plastic which fully or partly occludes the external ear (figure 1). The plastic used has a low melting temperature so that it may be moulded directly in the ear without causing burns. The plastic may be liquid or a pliable solid, like putty, when applied to the ear. On cooling the plastic hardens, retaining the shape of the external ear. The device may be formed as a simple plug to completely occlude the ear canal (figure 1) or have a channel formed in it (figure 2). There are many possible reasons for doing this, including, but not restricted to the channelling of sound into the ear or the attenuation of certain wavelengths of sound. The channel could be moulded, drilled, melted or formed using another method. Figure 1 shows the device occluding only the ear canal, but it could be adapted to occlude the whole or any part of the external ear.

An adaptor may be used to attach an object, for example, an electronic MP3 player earphone, to the device (figure 3). The adaptor would have a cup, claw or other adaptation on one end which has the form of the part of the object which is required to become permanently or temporarily attached to the lateral portion of the device. The proximal portion of the adaptor would be shaped with a flange, button, screw thread or other adaptation which would allow the adaptor to be permanently or reversibly attached to the device. There are a multitude of possible variations in the form of the adaptor.

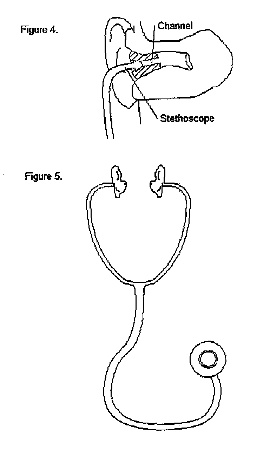

Alternatively an object may be attached to the device by moulding the device directly onto it (figure 4). An example would be the attachment of the arm of a stethoscope (figure 5), to which this method is particularly well suited, although it could also be used for earphones, radio equipment, audiovisual equipment or many other objects which are required to be attached to the device.

### Introduction to Drawings

Figure 1; the external ear with an occlusive device in place.
Figure 2; the external ear with an occlusive device in place, said occlusive device having a channel formed through the centre.
Figure 3; the external ear with an occlusive device in place, said occlusive device having a channel formed through the centre and an adaptor moulded into the lateral portion.
Figure 4; the external ear with an occlusive device in place, said occlusive device being moulded onto the flange on the arm of a stethoscope.
Figure 5; a stethoscope with the moulded earpieces attached.

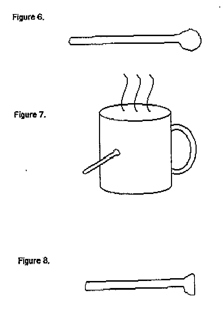

Figure 6; making a thermoplastic former used to mould the channel in an earpiece.
Figure 7; flattening of the end of the former against a warm mug.

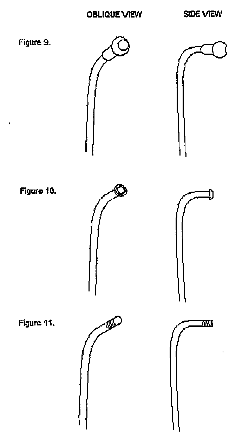

Figure 8; the finished former. Figure 9; a standard stethoscope earpiece.
Figure 10; a bevelled stethoscope arm with the earpiece removed.
Figure 11; a threaded stethoscope arm with the earpiece removed.

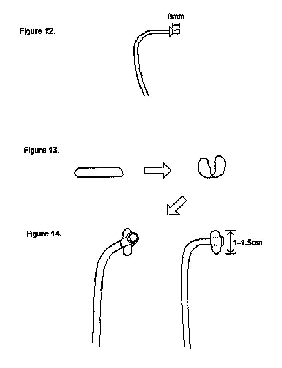

Figure 12; the stethoscope arm with the former in place.
Figures 13 and 14; making a flange to hold the new earpieces on.

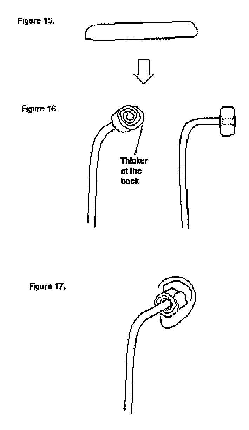

Figures 15 and 16; applying the thermoplastic to the shaft.
Figure 17; moulding the earpiece in the ear.

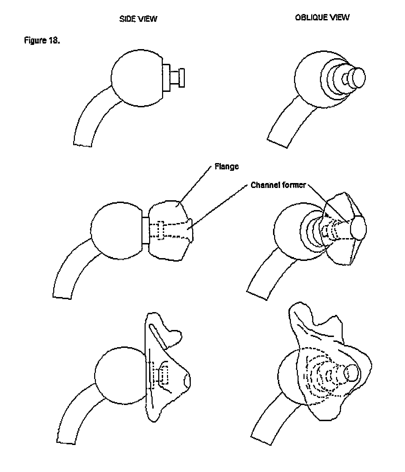

Figure 18; moulding the device directly onto an in-the-ear earphone.

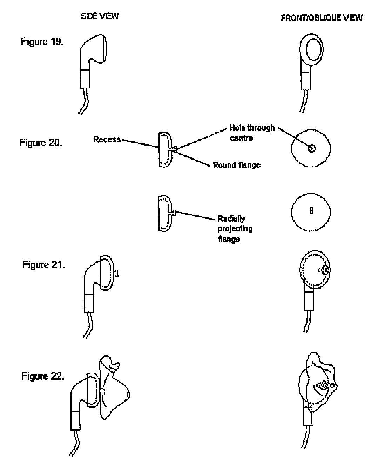

Figure 19; a typical in-the-ear earphone.
Figure 20; an example of an adaptor to attach the earphone to the occlusive device.
Figure 21; the adaptor attached to the earphone.
Figure 22; the adaptor attached to the earphone and the occlusive device.

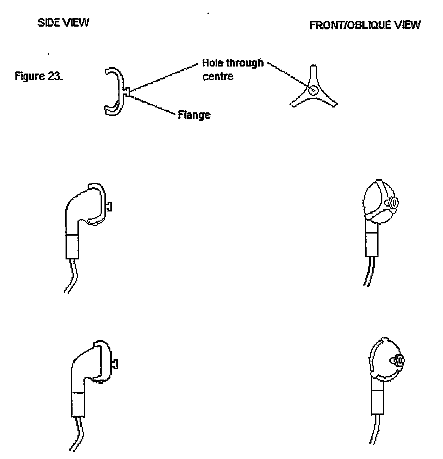

Figure 23; variations on the adaptor.

## Claims

1. A device which occludes the external ear made from a plastic with a melting point between 40 and 100 degrees centigrade.

2. A device according to claim 1 where the plastic is an aliphatic polyester.

3. A device according to claim 2 where the aliphatic polyester is polycaprolactone.

4. A device according to claim 1 with one or more materials or substances added.

5. A device according to claim 1 which occludes at least part of the external ear or ear canal.

6. A device according to claim 1 which only partially occludes the external ear or ear canal.

7. A device according to claim 1 where a channel is moulded, drilled or melted through the centre.

8. A device according to claim 1 used as an earpiece for electronic audio equipment.

9. A device according to claim 1 used as a stethoscope earpiece.

10. A device according to claim 1 used to connect an object to the ear.

11. A device according to claim 1 used as an earplug.

12. A device according to claim 1 where an object is inserted into the ear canal to mould the aliphatic polyester.

13. A device according to claim 1 used with an adaptor to facilitate attachment to another object.

14. Methods for the formation of a device according to claims 1 through to 13.
